(19) Europäisches Patentamt / European Patent Office / Office européen des brevets

(11) **EP 4 424 810 B1**

(12) **EUROPEAN PATENT SPECIFICATION**

(45) Date of publication and mention
of the grant of the patent:
**06.05.2026 Bulletin 2026/19**

(51) International Patent Classification (IPC):
**C12M 1/107** (2006.01)   **C12M 1/36** (2006.01)

(52) Cooperative Patent Classification (CPC):
**C12M 21/04; C12M 41/48**

(21) Application number: **23382186.7**

(22) Date of filing: **01.03.2023**

(54) **DIAGNOSING FAULTS DURING OPERATION OF AN ANAEROBIC DIGESTER**

DIAGNOSE VON FEHLERN WÄHREND DES BETRIEBS EINES ANAEROBEN FAULBEHÄLTERS

DIAGNOSTIC DE DÉFAUTS PENDANT LE FONCTIONNEMENT D'UN DIGESTEUR ANAÉROBIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**04.09.2024 Bulletin 2024/36**

(73) Proprietor: **Procycla SpA
9580000 Comuna Melipilla (CL)**

(72) Inventors:
• **DONOSO BRAVO, Andrés Eduardo**
**Comuna Viña del Mar (CL)**
• **SADINO RIQUELME, María Constanza**
**9250000 Comuna Maipú Región Metropolitana
(CL)**
• **VALDEBENITO ROLACK, Emky Héctor**
**4280395 Comuna Talcahuano (CL)**
• **HANSEN FERNÁNDEZ, Felipe Eduardo**
**7640274 Comuna De Vitacura (CL)**
• **GÓMEZ PASCUAL, Daniel**
**08241 Manresa (ES)**

(74) Representative: **ZBM Patents - Zea, Barlocci &
Markvardsen
Rambla de Catalunya, 123
08008 Barcelona (ES)**

(56) References cited:
WO-A1-2013/138690    WO-A1-2018/124383
WO-A1-2020/261151    WO-A1-2022/186980
US-A1- 2009 048 816

• **ERSAHIN MUSTAFA EVREN ED - MORENO
JAIME A ET AL: "Modeling the dynamic
performance of full-scale anaerobic primary
sludge digester using Anaerobic Digestion
Model No. 1 (ADM1)", BIOPROCESS AND
BIOSYSTEMS ENGINEERING, SPRINGER, DE,
vol. 41, no. 10, 12 July 2018 (2018-07-12), pages
1539 - 1545, XP036584924, ISSN: 1615-7591,
[retrieved on 20180712], DOI: 10.1007/
S00449-018-1981-5**
• **XUE LEI ET AL: "Nonlinear model predictive
control of anaerobic digestion process based on
reduced ADM1", 2015 10TH ASIAN CONTROL
CONFERENCE (ASCC), IEEE, 31 May 2015
(2015-05-31), pages 1 - 6, XP033197793, DOI:
10.1109/ASCC.2015.7244539**
• **ORDACE A ET AL: "Predictive control of
anaerobic digestion of wastewater sludge. A
feasibility study", 12 October 2012 (2012-10-12),
pages 1 - 7, XP055779751, ISBN:
978-1-4673-4534-7, Retrieved from the Internet
<URL:https://ieeexplore.ieee.org/stampPDF/
getPDF.jsp?tp=&arnumber=6379297&
ref=aHR0cHM6Ly9pZWVleHBsb3JlLmllZW
WUub3JnL2RvY3VtZW50LzYzNzkyOTc=>**
• **KAZEMI PEZHMAN ET AL: "Data-driven
techniques for fault detection in anaerobic
digestion process", PROCESS SAFETY AND
ENVIRONMENTAL PROTECTION, INSTITUTION
OF CHEMICAL ENGINEERS, RUGBY, GB, vol.
146, 24 December 2020 (2020-12-24), pages 905 -
915, XP086470349, ISSN: 0957-5820, [retrieved
on 20201224], DOI: 10.1016/J.PSEP.2020.12.016**

**Description**

The present disclosure is related to methods and systems for diagnosing faults during operation of an anaerobic digestion, e.g. faults in the anaerobic digestion process of the anaerobic digester, by employing the results of simulations of the anaerobic digestion process in a particular reversed engineering approach, using the output to detect input variations; and to anaerobic digestion systems in which these methods and systems are applied.

BACKGROUND

Anaerobic digestion is a widely used process in industrial and domestic waste management and production of fuels, such as biogas. Most commonly, anaerobic digesters are used for producing biogas, the digesters comprising a sealed vessel known as a reactor or digester, which may be designed and constructed in various shapes and sizes, in order to perform a substantially controlled anaerobic digestion process within. Generally, anaerobic processes may be slow and, when using a digester, they usually involve a lot of operator supervision, in order to keep the working conditions of the digester stable or within a specific range, to produce a desired amount of biogas.

Even though the anaerobic digestion process itself may be unpredictable or very prone to changes in the conditions in which it happens, there are systems which help predict the outcome of a digester (such as the production of biogas). Some of such systems use simulations of the process occurring in the digester using mathematical models of the anaerobic digestion (MM-AD in the following), such as the model known as Anaerobic Digestion Model No. 1 (ADM1), or others such as BIOmodel, AMOCO, etc., which models the operation of the digester, and are able to simulate the biogas output and other parameters over time during operation of the digester. More precisely, an MM-AD can simulate the steady state and dynamic behavior of an anaerobic digester. Traditionally, such models have been used to predict the biogas production of anaerobic digesters.

The diagnose of operation faults or malfunctions during operation of an anaerobic digester are currently performed only by reacting to measurements of the digester outputs on the basis of general references e.g. given in handbooks, best practice protocols, etc. Consequently, fault detection is not fast and efficient, and after a fault is diagnosed it may take several weeks before the anaerobic process returns to stable operation.

ERSAHIN MUSTAFA EVREN ED - MORENO JAIME A ET AL: "Modeling the dynamic performance of full-scale anaerobic primary sludge digester using Anaerobic Digestion Model No. 1 (ADM1)", BIOPROCESS ANO BIOSYSTEMS ENGINEERING, SPRINGER, DE, vol. 41, no. 10, 12 July 2018 (2018-07-12), pages 1539-1545 describes models that can be used to predict full-scale sludge digester performance. XUE LEI ET AL: "Nonlinear model predictive control of anaerobic digestion process based on reduced ADM1 ", 2015 10TH ASIAN CONTROL CONFERENCE (ASCC), IEEE, 31 May 2015 (2015-05-31), pages 1-6 describes an algorithm to control the anaerobic digestion process in biogas plants. ORDACE A ET AL: "Predictive control of anaerobic digestion of wastewater sludge. A feasibility study", 2012 16TH INTERNATIONAL CONFERENCE ON SYSTEM THEORY, CONTROL ANO COMPUTING (2012-10-12) pages 1-7 describes analysis, modeling, identification and predictive control of an anaerobic digestion process. Patent document WO 2013/138690 A1 describes photobioreactors for cultivation and/or propagation of a photosynthetic organism. Patent document US 2009/048816 A1 describes a method for online prediction of performance of a fermentation unit. KAZEMI PEZHAM ER AL: "Data-driven techniques for fault detection in an anaerobic digestion process", PROCESS SAFETY AND ENVIRONMENTAL PROTETCTION, INSTITUTION OF CHEMICAL ENGINEERS, RUGBY, GB, vol. 146, 24 December 2020 (2020-12-24), pages 905-915 describes a framework for detecting random faults in AD processes.

Thus, there is a need to detect and differentiate operation faults in anaerobic digesters in a sufficiently advanced manner over time, in order to enhance the prediction of malfunctions, thus minimizing the time of reaction and correction of said faults, maintaining a high productivity of biogas, and reducing the risk of idle times.

The inventors have now found that faults can be detected and anticipated by using a reverse engineering process to harness the outputs of an MM-AD simulation and assess the input and operational conditions that have caused those outputs, by comparing them with previously predicted reference values.

SUMMARY

According to an aspect of the present disclosure, a method for diagnosing operation faults during operation of an anaerobic digester is provided, comprising a preparation stage, wherein a mathematical model of anaerobic digestion (MM-AD) is run in a computing system. The preparation stage comprises:

- selecting an operation fault to be diagnosed;
- selecting an operational parameter OP-P that is associated with the selected operation fault; and
- running multiple simulations in the MM-AD to:

- select an output parameter OUT-P that varies with a variation of the operational parameter OP-P; and
- determine a threshold deviation of the output parameter OUT-P that indicates that the operation fault is occurring.

By an operational parameter "associated" with an operation fault it is meant that there is a known relationship between the parameter and the fault, such that it is known that the operational parameter varies when the fault occurs.

The preparation stage provides a fault condition, namely the threshold deviation of an output parameter indicating that the operation fault is occurring, and this fault condition is used in the subsequent digester operation stage, in order to diagnose and/or predict the operation fault.

The method may further comprise, after the preparation stage, a digester operation stage, wherein a mathematical model of anaerobic digestion (MM-AD) is run in a computing system, in parallel with the physical operation of the anaerobic digester, to simulate the operation of the digester over time. Such a digester operation stage comprises:

- inputting in the MM-AD the nominal values of the kinetic parameters and operational parameters of the digester;
- starting the operation of the digester and the MM-AD simulation;
- obtaining over time measured values MV of the output parameter OUT-P of the digester, using sensors and/or by performing sample analysis;
- obtaining over time expected values EV of the output parameter OUT-P, as provided by the MM-AD;
- comparing the measured values MV and the expected values EV of the output parameter OUT-P, over time;
- determining that the operation fault is occurring if the deviation between the measured value MV and the expected value EV of the output parameter OUT-P is above the threshold deviation determined in the preparation stage.

By "above the threshold deviation" it is meant that the deviation between the measured value MV and the expected value EV of the output parameter OUT-P is larger than the threshold deviation.

By running the MM-AD "in parallel" with the physical operation of the digester, it is meant that the operation of the digester and the MM-AD are started substantially at the same time. The same kinetic and operational parameters set in the digester are also inputted in the MM-AD, and therefore the MM-AD simulates the operation of the digester in real time, i.e. substantially "synchronized" with the digester.

By virtue of the data obtained in the preparation stage, and of the subsequent operation in parallel of the digester and the MM-AD, the claimed method allows diagnosing and/or predicting operation faults in an anaerobic digester by simply controlling the behavior of specific output parameters, e.g. output parameters which are easily measured e.g. with sensors and monitored during operation of the digester.

Thus, contrary to the known use of anaerobic digester models merely to predict the outputs (e.g. the biogas production) to be expected in the digester, the present disclosure provides methods in which the MM-AD simulations are used also to identify the variations of operational parameters that are causing a variation of a digester output, and diagnose operation faults that are associated with the variation of the operational parameters.

The present disclosure also relates to systems for diagnosing operation faults during operation of an anaerobic digester, and to anaerobic digester systems, according to the attached claims.

In particular, according to an aspect, the present disclosure relates to an anaerobic digester system, comprising:

- an anaerobic digester;
- a computing system with a mathematical model of anaerobic digestion (MM-AD), wherein in the digester operation stage the MM-AD is run in parallel with the physical operation of the anaerobic digester to simulate the operation of the digester over time;
- sensors, or a sample collection system, to obtain measurements of output parameters during operation of the anaerobic digester; and

- a processor to diagnose faults during a digester operation stage, the processor being configured to:

  - receive, over time, measured values of an output parameter of the digester;
  - receive from the MM-AD, over time, expected values of the output parameter;
  - compare over time the measured values and the expected values of the output parameter; and
  - determine that an operation fault is occurring if the deviation between the measured value and the expected value of the output parameter is above a predetermined threshold deviation.

In all embodiments of the present disclosure, the determination that an operation fault is occurring may cause the issue, e.g. by the processor, of a warning signal, a fault check report or status report, a data output, or other, such that operators in charge of the digester may be made aware of the potentially anomalous condition of the digestion process.

The present disclosure also relates to computer program products comprising instructions which, when the program is executed by a computer, cause the computer to carry out the steps of example methods disclosed herein and/or to control an anaerobic digester.

BRIEF DESCRIPTION OF THE DRAWINGS

Non-limiting examples of the present disclosure will be described in the following, with reference to the appended drawings, in which:

Figure 1 is a schematic diagram showing the main components of an anaerobic digester;

Figure 2 is a flowchart of a preparation stage according to an example of the method according to the present disclosure;

Figure 3 is a schematic graph for illustrating part of the preparation stage of Figure 2;

Figure 4 is a flowchart of a digester operation stage according to an example method according to the present disclosure;

Figure 5 is a schematic diagram showing the main components of an anaerobic digester operating according to an example method of the present disclosure; and

Figure 6 is a schematic graph for illustrating part of the preparation stage according to a specific example.

DESCRIPTION OF EMBODIMENTS

In operation of an anaerobic digester, a substrate is inputted in the digester inlet, and, while the anaerobic process is taking place within the digester reactor, an effluent (normally in liquid or semi-liquid form) and a gas (biogas, comprising different types of gases) is outputted through their respective outlets.

Furthermore, a digester usually comprises a plurality of interfaces (GUIs, operation buttons, etc....) which allow the supervision and operation of the digester by an operator, and may also comprise, for example, a plurality of sensors which may allow measuring different parameters related to the inlets, the outlets or other measurable variables within the digester, and/or a system for taking samples of the effluent, the biogas output, etc., and analyzing the samples in a laboratory.

According to the examples of the present disclosure, a first part of a method for diagnosing faults (hereinafter "preparation stage") may be performed before the operation of the anaerobic digester, and a second part (hereinafter "operation stage") may be performed while the operation of the digester is happening.

The first part of the method, i.e. the preparation stage, is performed in order to characterize one or more fault conditions of the digester and/or the digestion process: for instance, as will be described in detail below, as a result of this preparation stage it may be established that a certain deviation occurring in a certain parameter, with respect to an expected value, is associated with a specific operation fault of the digester. This preparation stage is performed offline, without the need of the digester itself being in operation, and using a computing system, typically running a mathematical model of anaerobic digestion (MM-AD), e.g. ADM1.

The second part of the method, i.e. the operation stage, involves actually diagnosing faults occurring in the anaerobic digester, as will be described in detail below, and is performed while the digester is in operation and the anaerobic digestion process is happening within the digester. The operation stage of the method also involves using a computing system running an MM-AD, e.g. ADM1, which may be the same model used for the first part.

An MM-AD model is fed with kinetic parameters and operational parameters of a specific anaerobic process, and simulates how the anaerobic digestion process is expected to behave during operation of the digester. Thus, when a simulation of an anaerobic digestion process of a specific anaerobic digester is performed using the MM-AD, the model provides expected values of the output parameters of the digester over time, i.e. it gives information on the expected performance and physical outputs of the digester within a future period of time, in case that no faults occur in the digester during operation.

The parameters of the digester used by an MM-AD model, e.g. ADM1, may be grouped as follows:

A) Kinetic parameters: parameters describing the properties of the digester itself, e.g., reaction capacity or decreasing capacity of the biomass inside the digester. For instance, depending on the MM-AD used, some parameters may be $k_h$ (hydrolysis constant, representing the capacity of the digester's microorganisms to degrade) and $B_0$ (representing how much biogas the digester can produce from the substrate feed in the digester). This type of parameters characterizes how the digester is, and they cannot be manipulated during the anaerobic process. For instance, when using ADM1, $k_h$ and $B_0$ are calibrated before performing a simulation, as known, taking into account features of the specific digester.

B) Operational parameters: parameters describing the inputs of the digester when in operation. More precisely, operational parameters may comprise parameters describing the physical inputs of the digester, such as substrate input flow (how much substrate is inputted in the digester), or the organic matter composition of the substrate; and operation-related parameters, such as the temperature set within the digester, the operation time set for the digester to operate, etc... This type of parameters characterizes how the digester operates, and they can be manipulated by the operator of the digester during the anaerobic process, for example, by setting operation-related parameters of the digester through a GUI, or by physically inputting more or less substrate at the inlet of the digester. Variations of one of these parameters while the anaerobic digestion process is being performed within the digester may cause faults or malfunctions in the digester.

C) Output parameters: parameters describing the output of the digester when in operation. More precisely, output parameters may comprise, for example, biogas output flow (or output of a specific type of a gas comprised within the biogas), effluent output flow, and the composition of the organic matter of the effluent, such as nitrogen content in the effluent, or volatile fatty acid content in the effluent. This type of parameters characterizes the performance of the digester, i.e., the output or result of the anaerobic process performed within the digester. Some of these parameters may be measured through sensors and/or via sample analysis, and/or monitored during the anaerobic process.

In the following, and in the attached figures, operational parameters will be indicated generally as OP-P, and output parameters will be indicated generally as OUT-P, and some examples of such parameters OP-P and OUT-P will be used: e.g. one OP-P may be the substrate input flow Qin, and one OUT-P may be the output biogas flow **BGout.**

During operation of an anaerobic digester, a variation of one or more specific operational parameters OP-P of the digester, with respect to the nominal values that are set for the operation of the digester, may be an indicator of an operation fault of the digester: for example, if there is a decrease in the substrate input flow Qin with respect to a nominal value of the substrate input flow, this may be caused by a "fault" in the digester, such as clogging of an inlet valve or conduit, or pump malfunction.

Furthermore, when an operational parameter OP-P varies during operation, this variation may cause the variation of one or more output parameters OUT-P of the digester: for example, if the substrate input flow Qin decreases, then the output biogas flow **BGout** also decreases. The level or value of some output parameters may change quickly, e.g. within minutes or hours from the variation of an operational parameter, while others may vary only slowly over time, e.g. it may take days to observe the variation of the output parameter OUT-P once the operational parameter OP-P has varied. On the other hand, for some combinations of OP-P and OUT-P the variation of the OUT-P may only be temporary, while for other combinations the variation may last over time.

Based on this, in the present invention, a particular reversed engineering analysis of the simulated outputs on an MM-AD is applied, by detecting a significant deviation of an output parameter OUT-P (e.g. output biogas flow BGout) with respect to an expected value, during operation of the digester, such a deviation allowing an operator to indirectly detect that an operational parameter OP-P (e.g. substrate input flow Qin) has changed in the digester, and therefore to determine that an operation fault associated with a change in the operational parameter (e.g. clogging of the inlet valve or conduit) has occurred, or may be occurring.

Example methods as disclosed herein may allow early detection of operation faults, such that smooth and efficient operation of the digester over time may be achieved, with satisfactory or optimum outputs, and idle times may be avoided.

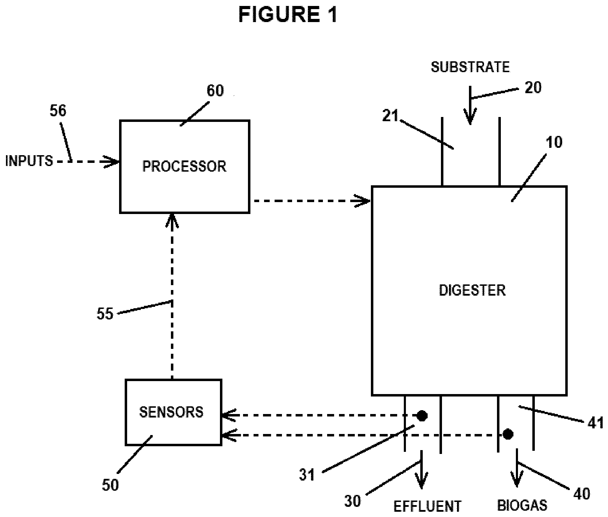

Figure 1 schematically illustrates a basic anaerobic digestion system in which methods according to the present disclosure may be performed. In Figure, 1, an anaerobic digester 10 is fed with a substrate input flow 20, typically rich in organic matter to be treated, through a digester inlet 21. An anaerobic process takes place within the digester, and a liquid or semi-liquid effluent flow 30 exits through a corresponding effluent outlet 31, while a biogas flow 40 exits through a corresponding gas outlet 41. A number of sensors 50 are arranged to take readings over time of several parameters of the digester and digestion process, including output parameters OUT-P related to the effluent and the biogas, such as e.g. temperature, pressure, flow rate, etc. Analysis and experimental tests may also be performed offline, i.e. in a laboratory and not in the anaerobic digester itself, by taking samples, e.g. of the effluent and/or biogas flow.

A processor 60, e.g. a microprocessor or CPU, may also be provided to receive inputs before and during the digester operation as described herein, e.g. sensor inputs 55 from sensors 50, and/or user inputs 56 regarding kinetic and/or operational parameters, or regarding results of offline analysis and tests; perform at least the operation stage of example methods disclosed herein for diagnosing faults during operation of the digester; and issuing alerts or warnings in any form, e.g. visual or audible alarms, computer output reports, or any other, when a potential operation fault is diagnosed.

The processor 60 may also provide periodic reports, e.g. daily, or reports upon user demand, with the diagnose of operation faults being contained in said reports.

The operator (i.e., a human operator) in charge of the anaerobic digester may receive such alerts or warnings, perform any suitable verifications, and decide to take any maintenance, repair, calibration, process adjustment step, or the like, towards a satisfactory or optimum operation of the anaerobic digestion process over time.

The preparation step may be performed using the same processor 60 or another, stand alone and/or remote computing system.

The first and second parts of a method according to the present disclosure (i.e. the preparation stage and the operation stage) are disclosed in more detail in the following.

**Preparation stage of the method: determining a fault condition based on the behavior of an output parameter**

The preparation stage of the method is performed, using an MM-AD, for:

- selecting at least an output parameter OUT-P, for example one that varies significantly with a variation of an operational parameter OP-P that is associated with an operation fault, and can be measured in the digester; and
- determining a threshold deviation of the output parameter OUT-P that indicates that the operation fault is occurring.

Thus, this preparation stage allows characterizing an operation fault of the digester as a function of the behavior of one or more output parameters OUT-P, which can be measured during the digester operation stage; more specifically, a behavior consisting in an excessive deviation from an expected value.

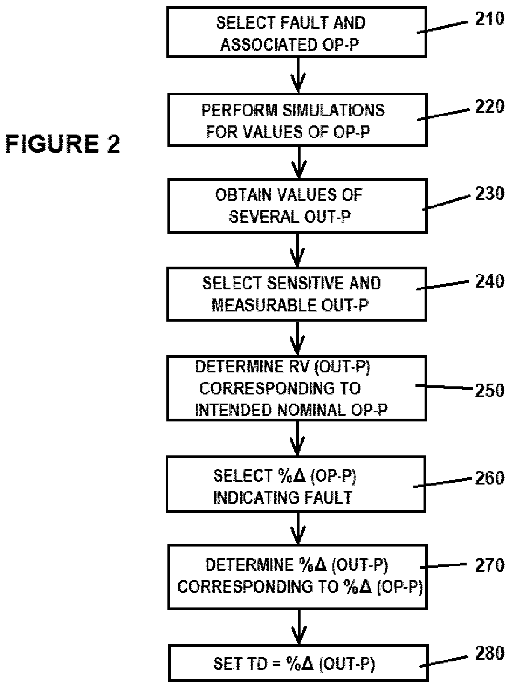

For instance, a preparation stage performed with an MM-AD, of an example method for diagnosing faults, may comprise, as illustrated in Figure 2:

- At 210, select an operation fault to be diagnosed, and an operational parameter OP-P associated with the fault;
- For this operational parameter OP-P that is known to vary when the fault occurs, multiple simulations are carried out at 220, for a set of values of the operational parameter OP-P, and a set of corresponding values of a number of output parameters OUT-P is determined at 230; and
- Based on the result of the simulations:

  ○ at least one output parameter OUT-P is selected at 240, that is sensitive to the variation of the operational parameter OP-P (i.e., that varies significantly with a variation of the operational parameter), and that can be measured (e.g. with sensors, and/or sample analysis) during operation of the digester; and
  ○ for a selected output parameter OUT-P:

    ▪ a reference value RV of the output parameter OUT-P is determined at 250, corresponding to an intended nominal value N of the operational parameter OP-P during the digester operation;
    ▪ at 260, a percentage variation %Δ(OP-P) of the operational parameter OP-P, with respect to the nominal value N, is selected, that is known to indicate an associated operation fault; and
    ▪ at 270, a percentage variation %Δ(OUT-P) of the output parameter OUT-P is determined, corresponding to the selected percentage variation %Δ(OP-P) of the operational parameter OP-P, and at 280 this percentage variation %Δ(OUT-P) is set as the threshold deviation TD of the output parameter OUT-P, with respect to the reference value RV, that indicates the operation fault is occurring.

By "nominal value" of an operational parameter, it is meant the value of the parameter that is established for the operation of the digester, and therefore also inputted in the MM-AD to simulate the operation of the digester.

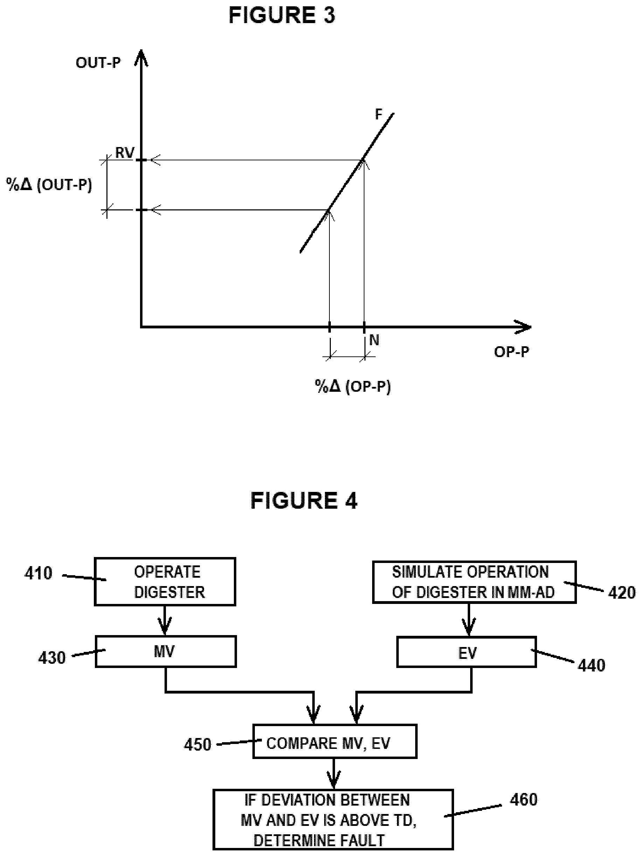

Figure 3 shows a graphical representation of how the threshold deviation TD of a selected output parameter OUT-P may be obtained, based on the simulations run in the MM-AD in the preparation stage. In Figure 3, N is the nominal value of OP-P set for the operation of the digester, and function F represents the variation of OUT-P with OP-P, as obtained from the simulations run in the MM-AD in this preparation phase (e.g. by linear regression); for the nominal value N of OP-P, there is a corresponding reference value RV of OUT-P, i.e. the expected value of OUT-P when the digester is operating correctly.

During the operation of the digester, the value of OP-P may undergo slight variations over time: OP-P may oscillate around the nominal value, and therefore OUT-P may also oscillate around the reference value RV, and this without a fault occurring. However, it is known, e.g. based on experimental and historical data and/or on the experience of a skilled operator, that a certain percentage variation %Δ(OP-P) of the operational parameter OP-P indicates an associated operation fault. For example, a 5% decrease of the substrate input flow with respect to the nominal value may indicate e.g. inlet clogging, or pump/valve malfunction.

This percentage variation %Δ(OP-P) of the operational parameter OP-P is employed to determine the corresponding percentage variation %Δ(OUT-P) of the output parameter OUT-P according to function F, as visible in the graph of Figure 2: for example, a variation of -20% (decrease). That is, when the value of the output parameter OUT-P varies from the reference value RV by -20%, this means that the operational parameter OP-P has varied -5%, and therefore the associated operation fault is occurring. The variation %Δ(OUT-P) of OUT-P is therefore a threshold deviation TD, and may be used as a fault condition.

During operation of the digester, if there is a deviation of the output parameter OUT-P, with respect to the expected reference value RV, that goes beyond the threshold deviation TD, this shows that the fault is occurring: therefore, the threshold deviation TD represents a "fault condition" that allows determining the operation fault is occurring. It is set as a percentage deviation with respect to the reference value RV of the output parameter OUT-P.

Once at least one fault condition has been determined for one operation fault, a look-up table with each operation fault and its corresponding fault condition may be stored in a database for subsequent use.

During the operation stage of the method, involving the physical operation of the digester and the MM-AD simulation running in parallel, the value of the operational parameter undergoes oscillations over time, within what is considered the normal operation of the digester, and so do multiple other operational parameters that influence the output parameters: the corresponding reference value RV for the output parameter OUT-P therefore also oscillates during operation. Therefore, during the operation stage of the method, the reference value RV for the output parameter OUT-P is not constant over time: it is the value of the output parameter OUT-P that is given by the MM-AD, i.e., the value that is expected or predicted in each moment, taking into account all the interactions that are considered by the MM-AD. The deviation of the measured values of the output parameter OUT-P with respect to the expected values given by the MM-AD is therefore monitored over time, and compared with the threshold deviation TD determined in the preparation stage (e.g. stored as a look-up table in a database, or in any other way), in order to establish if the fault condition is met and the fault is occurring.

On the other hand, the variation of an output parameter OUT-P as a function of an operational parameter OP-P may be different from lineal, and therefore in some cases extrapolating the threshold deviation TD to different nominal values of the OP-P may give poor results in diagnosing a fault. In some examples of the method, the preparation stage may involve running simulations in the MM-AD for obtaining several values of the threshold deviation TD, for different nominal values N of the operational parameter. For instance, a single preparation stage may be performed to obtain data for different nominal values of an operational parameter OP-P, and the relevant data may be later used in different digester operation stages, with different values of the nominal operational parameter OP-P.

The preparation stage may be performed for each operation fault to be diagnosed during operation of the digester with methods as disclosed herein, and for which there is one or more operational parameters that are known to vary as a consequence of the operation fault, or there is one or more operational parameters that are found to vary, by simulating different operation faults in the MM-AD.

The threshold deviation TD may be determined and applied for different time intervals: for example, in the preparation stage it may be determined for a single day, or for a time span of 5 consecutive days, or other, and it may be then applied accordingly to monitor faults during the operation stage.

Other kinds of fault conditions may also be envisaged, such as fault conditions based on more than one output parameter OUT-P, or based on a combination of output parameters and conditions.

## Operation stage of the method: diagnosing faults by monitoring an output parameter and comparing it with an expected value

After a preparation stage as described above and illustrated in Figures 2 and 3, in which, in relation to an operation fault to be diagnosed, an operational parameter OP-P and an output parameter OUT-P have been selected, and a threshold deviation TD has been determined, the digester operation stage of an example method for diagnosing faults comprises, as represented in Figure 4:

- The anaerobic digester is operated, at 410;
- In parallel with this physical operation of the digester, a mathematical model of anaerobic digestion (MM-AD) simulates the operation of the digester, at 420;
- Over time, during operation of the digester, measured values MV of the output parameter OUT-P are obtained through sensors and/or through results of experimental tests, at 430;
- In parallel with the operation of the digester, the MM-AD provides expected values EV of the output parameter OUT-P, at 440;
- The measured values MV and the expected values EV of the output parameter OUT-P are compared, over time, at 450; and
- At 460, if the deviation between MV and EV is above the percentage deviation threshold TD, as determined in the preparation stage, this indicates that the operational parameter OP-P has varied, and it is determined that the operation fault associated with the variation of the operational parameter OP-P, may be occurring in the digester. The operator in charge of the digester may then use this information to verify and control the digester as convenient.

It is understood that the kinetic and operational parameters of the digester are entered in the MM-AD before the beginning of the simulation.

The MM-AD runs in parallel with the physical operation of the digester: that is, the MM-AD simulates over time the operation of the digester, with the same nominal kinetic parameters and nominal operational parameters which are set for the digester; and it provides the values of the output parameters to be expected over time in the digester, assuming that the digester operates without faults and the kinetic and operational parameters remain substantially around their nominal values, with only minor oscillations.

It will be understood that examples of the method allow detecting and/or predicting operation faults in the digester simply by monitoring the behavior of specific output parameters, e.g. output parameters which are typically measured during operation, and without the need to measure or estimate, during the physical operation stage of the anaerobic digester and taking into account, variations that may have occurred in the nominal operational parameters that were set for the digester.

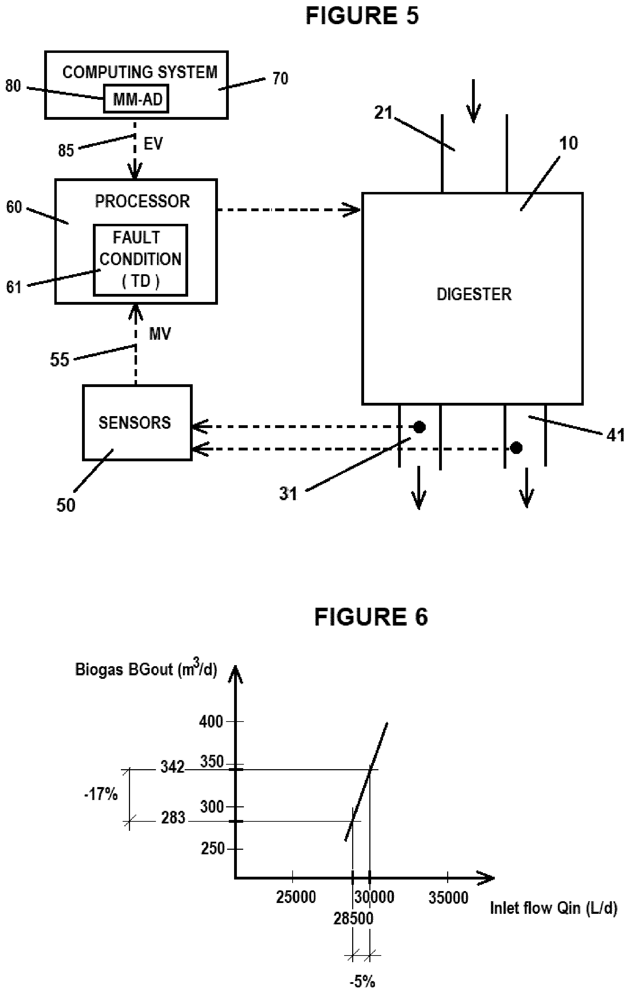

Figure 5 illustrates very schematically the main components of an anaerobic digester operating according to the method of Figure 4. In Figure 5, the anaerobic digester 10, as in Figure 1, is fed with a substrate through an inlet 21, and outputs effluent through an effluent outlet 31 and biogas through a gas outlet 41. Sensors 50 are arranged to take readings over time of output parameters OUT-P. Values of the output parameters OUT-P may also be obtained by taking samples e.g. of the effluent and/or biogas flow and analyzing the samples offline, e.g. in a laboratory.

The processor 60 receives readings 55 of the measured values MV of an output parameter OUT-P from the sensors 50 and/or from sample analysis, and also receives data 85 of the expected values EV of the output parameter OUT-P from a computing system 70 running an MM-AD 80 in parallel with the operation of the digester 10.

The processor 60 comprises a memory 61, in which a fault condition (namely, the threshold deviation TD for output parameter OUT-P determined in the preparation stage) has been previously stored. Over time, the processor 60 compares the deviation between the expected values EV and measured values MV, as in method step 450 of Figure 4; and based on the comparison determines if the fault is occurring, as in method step 460 of Figure 4.

The preparation stage and digester operation stage are not necessarily performed close in time one after the other, and the two stages are not necessarily paired: (i.e. one preparation stage before each intended digester operation stage according to methods disclosed above). Furthermore, the same computing system may be used in both stages of the method; alternatively, different computing systems may be used in each stage.

Indeed, there is generally no need to perform a preparation stage every time a digester is going to be put in operation (i.e. for every digester operation stage). It may be sufficient to perform a preparation stage to obtain fault conditions for multiple values of kinetic parameters and operational parameters of a digester, and use the obtained fault conditions in a subsequent digester operation stage, in any digesters having the physical structure, dimensions, kinetic and operational parameters, etc., used in the preparation stage.

According to the present disclosure it is foreseen to perform a preparation stage for multiple fault conditions, operational parameters, and specific nominal values of an anaerobic digester kinetic and operational parameters, and store the resulting data obtained to characterize the fault conditions (e.g., threshold deviations for the relevant output parameter(s) for each operation fault), and subsequently use the stored data by performing only the operation stage of the described method, in different anaerobic digesters.

**EXAMPLE 1**

A particular, non-limiting and simple example of the method, applied to a particular operation fault and to a particular operational parameter and output parameter, will be described in detail in the following.

In this example, the operational parameter OP-P is the substrate input flow **Qin,** which is set at a nominal value for the operation of the digester, and which may suffer an undesired variation over time, if the substrate inlet clogs or the pump malfunctions. An operation fault associated with **Qin** is therefore a substrate inlet fault, due e.g. to clogging or a pump or valve malfunction.

In the following, an example will be described in detail of how a fault condition is determined, and then applied to detect this fault during the anaerobic digester operation, by taking advantage of the known relationship between the fault and the substrate input flow **Qin.**

**Preparation stage**

- Selecting a suitable output parameter

Kinetic parameters of the Anaerobic Digestion Model No.1 (ADM1), which was chosen as the MM-AD representing the process of an anaerobic digester in which faults are to be diagnosed, are first calibrated, as already known, such as $k_h$ and $B_0$.

Simulations are then performed with the ADM1 by varying operational parameters, in this case at least the substrate input flow **Qin,** for a set of values around the nominal value to be employed in the operation of the digester, and differing from this nominal value; in the simulations, corresponding values of several output parameters (see tables below) are obtained for each value of the substrate input flow **Qin.**

The nominal value of the substrate input flow **Qin** in this example is set to 30000 L/d (liters/day), i.e. 30 $m^3$/d.

The results of the simulations are partially shown in the following tables 1 to 8.

TABLE 1

| Qin (L/d) | $Q_{in}$ variation | $N_{org}$ (gN/L) | $N_{org}$ variation |
|---|---|---|---|
| 45000 | 50% | 1,035 | 9% |
| 31500 | 5% | 0,957 | 1% |
| 30600 | 2% | 0,951 | 0% |
| **30000** | | 0,947 | |
| 29400 | -2% | 0,942 | 0% |
| 28500 | -5% | 0,937 | -1% |
| 0 | | 0,922 | -3% |

TABLE 2

| Qin (L/d) | $Q_{in}$ variation | pH | pH variation |
|---|---|---|---|
| 45000 | 50% | 6,870 | -1% |
| 31500 | 5% | 6,939 | 0% |
| 30600 | 2% | 6,944 | 0% |
| **30000** | | 6,947 | |
| 29400 | -2% | 6,951 | 0% |
| 28500 | -5% | 6,962 | 0% |
| 0 | | 6,977 | 0% |

TABLE 3

| Qin (L/d) | $Q_{in}$ variation | $COD_{total}$ (gCODIL) | $COD_{total}$ variation |
|---|---|---|---|
| 45000 | 50% | 29,1 | 4% |
| 31500 | 5% | 28,1 | 0% |
| 30600 | 2% | 28,1 | 0% |
| **30000** | | 28,0 | |
| 29400 | -2% | 28,0 | 0% |
| 28500 | -5% | 27,9 | 0% |
| 0 | | 27,7 | -1% |

TABLE 4

| Qin (L/d) | $Q_{in}$ variation | $BM_{met}$ (gCODIL) | $BM_{met}$ variation |
|---|---|---|---|
| 45000 | 50% | 0,5405 | 20% |

(continued)

| Qin (L/d) | $Q_{in}$ variation | $BM_{met}$ (gCODIL) | $BM_{met}$ variation |
|---|---|---|---|
| 31500 | 5% | 0,4617 | 3% |
| 30600 | 2% | 0,4550 | 1% |
| **30000** | | 0,4497 | |
| 29400 | -2% | 0,4445 | -1% |
| 28500 | -5% | 0,4389 | -2% |
| 0 | | 0,4259 | -5% |

TABLE 5

| Qin (L/d) | $Q_{in}$ variation | Biogas (m³/d) | Biogas variation |
|---|---|---|---|
| 45000 | 50% | 567 | 66% |
| 31500 | 5% | 388 | 14% |
| 30600 | 2% | 365 | 7% |
| **30000** | | **342** | |
| 29400 | -2% | 313 | -8% |
| **28500** | **-5%** | **283** | **-17%** |
| 0 | | 122 | -64% |

TABLE 6

| Qin (L/d) | $Q_{in}$ variation | Methane (m³/d) | Methane variation |
|---|---|---|---|
| 45000 | 50% | 216 | 79% |
| 31500 | 5% | 145 | 20% |
| 30600 | 2% | 133 | 10% |
| **30000** | | **121** | |
| 29400 | -2% | 105 | -13% |
| 28500 | -5% | 89,1 | -26% |
| 0 | | 22,2 | -82% |

TABLE 7

| Qin (L/d) | $Q_{in}$ variation | Aac (gCODIL) | Aac variation |
|---|---|---|---|
| 45000 | 50% | 0,0036 | 192% |
| 31500 | 5% | 0,0014 | 14% |
| 30600 | 2% | 0,0013 | 7% |
| **30000** | | **0,0012** | |
| 29400 | -2% | 0,0011 | -7% |
| 28500 | -5% | 0,0010 | -15% |
| 0 | | 0,0008 | -35% |

TABLE 8

| Qin (L/d) | $Q_{in}$ variation | $H_2$ (gCOD/L) | $H_2$ variation |
|---|---|---|---|
| 45000 | 50% | 72,2 | 39% |

(continued)

| Qin (L/d) | $Q_{in}$ variation | $H_2$ (gCOD/L) | $H_2$ variation |
|---|---|---|---|
| 31500 | 5% | 53,9 | 4% |
| 30600 | 2% | 52,7 | 2% |
| **30000** | | **51,9** | |
| 29400 | -2% | 51,1 | -2% |
| 28500 | -5% | 49,9 | -4% |
| 0 | | 30,3 | -42% |

The results of the simulations shown in tables 1-4 show that some output parameters, such as Organic Nitrogen $N_{org}$ (TABLE 1), pH (TABLE 2), Total Chemical Oxygen Demand $COD_{total}$ (TABLE 3), or Methanogenic Biomass $BM_{met}$ (TABLE 4), undergo very small variations with a variation of $Q_{in}$, e.g. a 20% variation, or lower, with an increase of 50% of $Q_{in}$; they are therefore not useful for detecting a variation of $Q_{in}$, and are therefore discarded, and not employed in the method for diagnosing faults.

On the contrary, the results of the simulations shown in tables 5-7 show that other output parameters, such as Biogas (TABLE 5), Methane (TABLE 6) or Acetic Acid **Aac** (TABLE 7), undergo significant variations, e.g. a 66% variation, or higher, with an increase of 50% of $Q_{in}$, and are therefore highly sensitive. Furthermore, these three parameters are commonly measured during the operation of the anaerobic digester, and are therefore useful as output parameters to be employed in diagnosing faults as disclosed herein.

The results of the simulation for Hydrogen $H_2$ are in TABLE 8: as shown by the results, this output parameter may be considered quite sensitive, and potentially useful as an output parameter for diagnosing faults: however, Hydrogen output is not usually measured in anaerobic digestion plants, and it is therefore discarded in this example.

In this example, and on the basis of the results of the simulation, biogas flow output **(BGout** in the following) is selected as the output parameter OUT-P to be employed, because it shows a significant variation with **Qin**, and is also an output parameter that is typically measured during the physical operation of anaerobic digesters.

- Determining the reference value and threshold deviation

As explained above, the reference value for the output parameter **BGout** is the value given by the ADM1 for **BGout** when the value of **Qin** is the intended nominal value, i.e. the value of **Qin** at which the anaerobic digester is planned or intended to be operated.

For example, for nominal **Qin** of 30000 L/d, the ADM1 simulation may give a reference value RV of 342 m$^3$/d for **BGout,** as shown in TABLE 5 and in Figure 6, which is similar to Figure 3 discussed above.

Based e.g. on experimental tests carried out in physical operation of anaerobic digesters, and/or on the knowledge of experienced operators, it is known that, in general, a certain variation (decrease) of **Qin** with respect to the nominal value occurs when there is a certain degree of clogging of the substrate inlet, this variation of $Q_{in}$ being:

$$\%\Delta(Qin) = -5\%$$

As shown by the results of the simulations in TABLE 5, and as graphically represented in Figure 6, it is determined in this example that such a decrease of 5% of Qin with respect to the nominal value gives a deviation (decrease) of BGout with respect to the reference value of:

$$\%\Delta(BGout) = -17\%$$

Thus, at the end of the preparation stage, it is determined that the percentage threshold deviation of the output parameter BGout that indicates the substrate inlet is clogging is:

$$TD = -17\%$$

In other words, if during operation of the digester a measured value of the biogas flow output (in the following, $BGout_{MV}$, obtained from sensors or from the analysis of samples of the biogas output, or both) decreases beyond the threshold deviation TD of 17% from the expected value (in the following, $BGout_{EV}$, obtained by the ADM1 that is running in parallel), this deviation indicates that the substrate inlet may be clogging, or an inlet pump/valve may be malfunctioning.

It will be understood that the fault condition may be expressed as a percentage deviation, i.e.,

$$\frac{BGout_{EV} - BGout_{MV}}{BGout_{EV}} \times 100 > 17\%$$

and may equally be expressed as:

$$\frac{BGout_{EV} - BGout_{MV}}{BGout_{EV}} > 0.17$$

This fault condition will be applied in the subsequent operation stage of the method, as will be described below.

In other examples, a different output parameter may be selected to diagnose a fault condition; or more than one output parameter may be selected, e.g. for better accuracy in the diagnostic and/or to distinguish between different operational faults.

A preparation process such as described for **Qin, BGout** may also be carried out for other operation faults of the digester, for instance using other operational parameters OP-P, and/or other output parameters OUT-P, and obtaining corresponding fault conditions.

For example, other operation faults that may be diagnosed with methods according to this disclosure are drops in the temperature due to failures of the heating system, or variations in the organic load of the substrate, such as higher organic load concentration than expected, e.g. due to errors in the laboratory measures, or to undetected dilutions of the organic load.

Furthermore, several fault conditions, based on different output parameters OUT-P, or on combinations of several output parameters OUT-P, may be used to determine the same operation fault of the digester (e.g. clogging of the substrate inlet); combinations of fault conditions, e.g. of two or more output parameters OUT-P, may also be used to diagnose with more accuracy the degree and/or the reason behind an operation fault (e.g. the degree of clogging of the inlet).

## Operation stage

In the subsequent operation stage, i.e. a production stage, the digester is operated with **Qin** at or around a nominal value of 30000 L/d.

A simulation using the ADM1 is also operated, in parallel with the digester, i.e. starting at the same time, and with the same kinetic and operational parameters as the physical digester, including **Qin** at nominal value of 30000 L/d, and with the kinetic parameters $k_h$ and $B_0$ calibrated, as known in the art and as described above for the preparation stage.

Over time, e.g. every predetermined number of hours, or daily, or other, successive measured values $BGout_{MV}$ of the biogas flow **BGout** are obtained, and successive expected or predicted values $BGout_{EV}$ are obtained from the ADM1, and the deviation of the measured value with respect to the predicted value is calculated, using the formula:

$$BGout\ deviation = \frac{BGout_{EV} - BGout_{MV}}{BGout_{EV}}$$

It will be understood that the calculation is made with corresponding measured and predicted values: i.e., the pair of measured value and predicted value in the above formula both refer to substantially the same moment of operation of the digester.

If this deviation is above the threshold deviation TD obtained in the preparation stage, then the presence of clogging or of a pump/valve malfunction at the substrate inlet of the digester is determined.

In other words, the fault condition is diagnosed by using the fault condition obtained in the preparation stage above, and a substrate inlet clogging fault may be diagnosed if:

$$\frac{BGout_{EV} - BGout_{MV}}{BGout_{EV}} > 0.17$$

The same process may be done for multiple other output parameters OUT-P and operational faults, provided the corresponding threshold deviations have been predetermined in the preparation stage.

As will be apparent from the above description, after the preparation stage is performed, the method does not require measuring or estimating the values of operational parameters: it allows diagnosing faults simply by measuring some output parameters - which are already measured in most anaerobic digesters - and running an MM-AD simulation in parallel with the operation of the digester.

OTHER EXAMPLES

Other operation fault conditions may be diagnosed using a method according to the present disclosure.

By way of example, further operation faults, a corresponding set of associated operational parameters, which have been found to vary when that fault occurs, and the corresponding fault conditions (based on output parameters that may be measured by sensors and/or by sample analysis during operation of the digester), that were derived by performing a preparation stage of a method according to the present disclosure, are shown in the following.

In the following formulae, the operation faults characterized by respective fault conditions, obtained by a preparation process as described above (e.g. including the results of simulations such as shown in TABLE 5, TABLE 6 and TABLE 7, and results of further similar simulations), are:

- Drop of internal temperature of the digester;
- Organic Overload (either produced by an increased flow rate at the substrate inlet of the digester, or by a wrong substrate dilution of the substrate); and
- Clogging or reduced flow rate at the input of the digester.

**Operation fault: Drop of internal temperature of the digester**

Associated Operational Parameter: Operation temperature

Output Parameters fault condition:

$$\frac{Biogas_{EV} - Biogas_{MV}}{Biogas_{EV}} > 0.25$$

AND

$$\frac{Methane_{EV} - Methane_{MV}}{Methane_{EV}} > 0.20$$

**Operation fault: Organic Overload**

Associated Operational Parameters: Substrate inlet flow and substrate characterization

Output Parameters fault condition:

$$\frac{Biogas_{MV} - Biogas_{EV}}{Biogas_{EV}} > 0.14$$

AND

$$\frac{Methane_{MV} - Methane_{EV}}{Methane_{EV}} > 0.20$$

Wherein the overload is considered associated to an increase in the substrate inlet flow if, additionally:

$$\frac{Aac_{MV} - Aac_{EV}}{Aac_{EV}} > 0.14$$

Otherwise, overload may be caused by a wrong substrate dilution (substrate characterization).

**Operation fault: Decrease in the Organic Load**

Associated Operational Parameter: Substrate inlet flow

Output Parameters fault condition:

$$\frac{Biogas_{EV} - Biogas_{MV}}{Biogas_{EV}} > 0.17$$

AND

$$\frac{Methane_{EV} - Methane_{MV}}{Methane_{EV}} > 0.26$$

Wherein the decrease in organic load is considered associated to a decrease in the substrate inlet flow if, additionally:

$$\frac{Aac_{EV} - Aac_{MV}}{Aac_{EV}} > 0.15$$

And wherein the decrease in organic load is considered associated to clogging if:

$$\frac{Biogas_{EV} - Biogas_{MV}}{Biogas_{EV}} > 0.59$$

AND

$$\frac{Methane_{EV} - Methane_{MV}}{Methane_{EV}} > 0.77$$

AND

$$\frac{Aac_{EV} - Aac_{MV}}{Aac_{EV}} > 0.30$$

In all the above formulae:

- *Biogas* is the biogas output flow;
- *Methane* is the methane output flow;
- *Aac* is the content of Acetic Acid in the effluent;
- The subindex *EV* indicates an *Expected Value* (given by the MM-AD);
- The subindex *MV* indicates a *Measured Value* (obtained from sensor readings and/or sample analysis).

For reasons of completeness, various aspects of methods according to the present disclosure are set out in the following clauses:

Clause 1. A method for diagnosing operation faults during operation of an anaerobic digester, comprising:

- a digester operation stage, wherein a mathematical model of anaerobic digestion (MM-AD) is run in a computing system, in parallel with the physical operation of an anaerobic digester, to simulate the operation of the digester over time, the digester operation stage comprising:

  - inputting in the MM-AD the nominal values of the kinetic parameters and operational parameters of the digester;

- starting the operation of the digester and the MM-AD simulation;
- obtaining over time measured values MV of an output parameter OUT-P of the digester, using sensors and/or sample analysis;
- obtaining over time expected values EV of the output parameter OUT-P, as provided by the MM-AD;
- comparing the measured values MV and the expected values EV of the output parameter OUT-P, over time;
- determining that an operation fault is occurring if the deviation between the measured value MV and the expected value EV of the output parameter OUT-P is above a predetermined threshold deviation.

Clause 2. A method according to clause 1, further comprising:

- running a preparation stage before the operation stage, the preparation stage comprising:

  - selecting an operation fault to be diagnosed;
  - selecting an operational parameter OP-P that is associated with the selected operation fault; and
  - running multiple simulations using a mathematical model of anaerobic digestion (MM-AD) run in a computing system, to:

    - select an output parameter OUT-P that varies with a variation of the operational parameter OP-P; and
    - determine a threshold deviation of the output parameter OUT-P that indicates that the operation fault is occurring; and

- using said threshold deviation of the output parameter as the predetermined threshold deviation in the subsequent operation stage of the method.

Clause 3. A system for diagnosing operation faults during operation of an anaerobic digester, the system comprising:

- a computing system with a mathematical model of anaerobic digestion (MM-AD), and

- a processor configured to diagnose faults during a digester operation stage, wherein in the digester operation stage the MM-AD is run in parallel with the physical operation of an anaerobic digester to simulate the operation of the digester over time, the processor being configured to:

  - receive, over time, measured values MV of an output parameter OUT-P of the digester;
  - receive from the MM-AD, over time, expected values EV of the output parameter OUT-P;
  - compare over time the measured values MV and the expected values EV of the output parameter OUT-P; and
  - determine that an operation fault is occurring if the deviation between the measured value MV and the expected value EV of the output parameter OUT-P is above a predetermined threshold deviation.

Examples of methods and systems according to any of these clauses may further comprise any combination of steps and/or features foreseen in the attached set of claims.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

Furthermore, although the examples described with reference to the drawings comprise computing apparatus/systems and processes performed in computing apparatus/systems, the invention also extends to computer programs, particularly computer programs on or in a carrier, adapted for putting the system into practice.

**Claims**

1. A method for diagnosing operation faults during operation of an anaerobic digester (10), comprising:

   - a preparation stage, wherein a mathematical model of anaerobic digestion (MM-AD) is run in a computing system, the preparation stage comprising:

     - selecting (210) an operation fault to be diagnosed;

- selecting (210) an operational parameter OP-P that is associated with the selected operation fault; and
- running (220) multiple simulations in the MM-AD to:

  - select (230, 240) an output parameter OUT-P that varies with a variation of the operational parameter OP-P; and
  - determine (250, 260, 270, 280) a threshold deviation of the output parameter OUT-P that indicates that the operation fault is occurring; and

after the preparation stage:
- a digester operation stage, wherein a mathematical model of anaerobic digestion (MM-AD) is run in a computing system, in parallel with the physical operation of the anaerobic digester, to simulate the operation of the digester over time, the digester operation stage comprising:

  - inputting in the MM-AD nominal values of kinetic parameters and operational parameters of the digester;
  - starting (410, 420) the operation of the digester and the MM-AD simulation;
  - obtaining (430) over time measured values MV of the output parameter OUT-P of the digester, using sensors and/or by performing sample analysis;
  - obtaining (440) over time expected values EV of the output parameter OUT-P, as provided by the MM-AD;
  - comparing (450) the measured values MV and the expected values EV of the output parameter OUT-P, over time;
  - determining (460) that the operation fault is occurring if the deviation between the measured value MV and the expected value EV of the output parameter OUT-P is above the threshold deviation determined in the preparation stage.

2. A method according to claim 1, wherein the preparation stage comprises selecting more than one operation fault to be diagnosed and, for each operation fault:

  - selecting (210) an operational parameter OP-P that is associated with the selected operation fault; and
  - running (220) multiple simulations in the MM-AD, to:

  - for each selected operational parameter OP-P, select (230, 240) an output parameter OUT-P that varies with a variation of the corresponding operational parameter OP-P; and
  - for each selected output parameter OUT-P, determine (250, 260, 270, 280) a threshold deviation that indicates that the selected operation fault is occurring.

3. A method according to claim 2, further comprising, after the preparation stage:

  - a digester operation stage, wherein a mathematical model of anaerobic digestion (MM-AD) is run in a computing system, in parallel with the physical operation of the anaerobic digester, to simulate the operation of the digester over time, the digester operation stage comprising:

  - inputting in the MM-AD the nominal values of the kinetic parameters and operational parameters of the digester;
  - starting (410, 420) the operation of the digester and the MM-AD simulation;
  - obtaining (430) over time measured values MV of the output parameter OUT-P of the digester, using sensors and/or by performing sample analysis;
  - obtaining (440) and comparing (450) over time measured values MV and expected values EV of each selected output parameter OUT-P, and
  - determining (460) that one of the selected operation faults is occurring if the deviation between the measured value MV and the expected value EV of one of the selected output parameters OUT-P is above the corresponding threshold deviation determined in the preparation stage.

4. A method according to any one of the preceding claims, wherein the preparation stage comprises running (220) multiple simulations in the MM-AD to determine threshold deviations of the output parameter OUT-P that indicate that the operation fault is occurring, for at least two different time periods.

5. A method according to claim 4, wherein threshold deviations of the output parameter OUT-P that indicate that the operation fault is occurring, are determined for a time period of one day.

6. A method according to claim 4, wherein threshold deviations of the output parameter OUT-P that indicate that the operation fault is occurring, are determined for a time period of several consecutive days.

7. A method according to any one of the preceding claims, wherein selecting an output parameter OUT-P that varies with a variation of the operational parameter comprises performing a sensitivity analysis of output parameters.

8. A method according to claim 7, wherein the sensitivity analysis comprises:

- selecting several output parameters that may vary with a variation of the operational parameter;
- simulating the variation of each output parameter, over a predetermined period of time, when the operational parameter varies with respect to a nominal value to be employed in the digester operation; and
- comparing the variations over the predetermined period of time of the plurality of output parameters, for the same variation of the operational parameter; and
- selecting at least one output parameter for which the variation over time is higher than for other output parameters.

9. A method according to any one of the preceding claims, wherein the preparation stage comprises calibrating the kinetic parameters of the MM-AD of an anaerobic digester in which faults are to be diagnosed.

10. A method according to any one of the preceding claims, wherein the operation stage comprises calibrating the kinetic parameters of the MM-AD of the anaerobic digester.

11. A method according to any one of the preceding claims, wherein at least one selected output parameter is the biogas output flow of the anaerobic digester, and/or the methane output flow of the anaerobic digester.

12. A system for diagnosing operation faults during operation of an anaerobic digester (10), the system comprising:

- a computing system (70) with a mathematical model of anaerobic digestion (MM-AD), and
- a processor (60) configured to perform the method of any of claims 1 to 11.

13. An anaerobic digester system, comprising:

- an anaerobic digester (10);
- sensors (50), and/or a sample collection system, to obtain measurements of output parameters during operation of the anaerobic digester (60); and
- a system for diagnosing operation faults during operation of the anaerobic digester, according to claim 12.

**Patentansprüche**

1. Ein Verfahren zum Diagnostizieren von Betriebsfehlern während des Betriebs eines anaeroben Faulbehälters (10), umfassend:

- eine Vorbereitungsstufe, wobei ein mathematisches Modell der anaeroben Faulung (MM-AD) in einem Computersystem ausgeführt wird, wobei die Vorbereitungsstufe Folgendes umfasst:

- auswählen (210) eines zu diagnostizierenden Betriebsfehlers;
- auswählen (210) eines Betriebsparameters OP-P, der dem ausgewählten Betriebsfehler zugeordnet ist; und
- ausführen (220) mehrerer Simulationen im MM-AD, um:

- einen Ausgangsparameter OUT-P auszuwählen (230, 240), der mit einer Variation des Betriebsparameters OP-P variiert; und
- eine Schwellenwertabweichung des Ausgangsparameters OUT-P zu bestimmen (250, 260, 270, 280), die angibt, dass der Betriebsfehler auftritt; und

nach der Vorbereitungsphase:
- eine Faulbehälterbetriebsstufe, wobei ein mathematisches Modell der anaeroben Faulung (MM-AD) in einem Computersystem parallel zum physikalischen Betrieb des anaeroben Faulbehälters ausgeführt wird, um den Betrieb des Faulbehälters über die Zeit zu simulieren, wobei die Faulbehälterbetriebsstufe Folgendes umfasst:

- eingeben der MM-AD-Nennwerte von kinetischen Parametern und Betriebsparametern des Faulbehälters;
- starten (410, 420) des Betriebs des Faulbehälters und der MM-AD-Simulation;
- erhalten (430) von über die Zeit gemessenen Werten MV des Ausgangsparameters OUT-P des Faulbehälters unter Verwendung von Sensoren und/oder durch Durchführen einer Probenanalyse;
- erhalten (440) von über die Zeit erwarteten Werten EV des Ausgangsparameters OUT-P, wie durch das MM-AD bereitgestellt;
- vergleichen (450) der gemessenen Werte MV und der erwarteten Werte EV des Ausgangsparameters OUT-P über die Zeit;
- bestimmen (460), dass der Betriebsfehler auftritt, wenn die Abweichung zwischen dem gemessenen Wert MV und dem erwarteten Wert EV des Ausgangsparameters OUT-P über der Schwellenwertabweichung liegt, die in der Vorbereitungsstufe bestimmt worden ist.

2. Ein Verfahren nach Anspruch 1, wobei die Vorbereitungsstufe das Auswählen von mehr als einem zu diagnostizierenden Betriebsfehler und ,für jeden Betriebsfehler, Folgendes umfasst:

- auswählen (210) eines Betriebsparameters OP-P, der dem ausgewählten Betriebsfehler zugeordnet ist; und
- ausführen (220) mehrerer Simulationen im MM-AD, um:

- für jeden ausgewählten Betriebsparameter OP-P einen Ausgangsparameter OUT-P auszuwählen (230, 240), der mit einer Variation des entsprechenden Betriebsparameters OP-P variiert; und
- für jeden ausgewählten Ausgangsparameter OUT-P eine Schwellenwertabweichung zu bestimmen (250, 260, 270, 280), die angibt, dass der ausgewählte Betriebsfehler auftritt.

3. Ein Verfahren nach Anspruch 2, ferner umfassend nach der Vorbereitungsstufe:

- eine Faulbehälterbetriebsstufe, wobei ein mathematisches Modell der anaeroben Faulung (MM-AD) in einem Computersystem parallel zum physikalischen Betrieb des anaeroben Faulbehälters ausgeführt wird, um den Betrieb des Faulbehälters über die Zeit zu simulieren, wobei die Faulbehälterbetriebsstufe Folgendes umfasst:

- eingeben der Nennwerte der kinetischen Parameter und Betriebsparameter des Faulbehälters in das MM-AD;
- starten (410, 420) des Betriebs des Faulbehälters und der MM-AD-Simulation;
- erhalten (430) von über die Zeit gemessenen Werten MV des Ausgangsparameters OUT-P des Faulbehälters unter Verwendung von Sensoren und/oder durch Durchführen einer Probenanalyse;
- erhalten (440) und Vergleichen (450) von über die Zeit gemessenen Werten MV und erwarteten Werten EV jedes ausgewählten Ausgangsparameters OUT-P, und
- bestimmen (460), dass einer der ausgewählten Betriebsfehler auftritt, wenn die Abweichung zwischen dem gemessenen Wert MV und dem erwarteten Wert EV eines der ausgewählten Ausgangsparameter OUT-P über der entsprechenden Schwellenwertabweichung liegt, die in der Vorbereitungsstufe bestimmt worden ist.

4. Ein Verfahren nach einem der vorhergehenden Ansprüche, wobei die Vorbereitungsstufe das Ausführen (220) mehrerer Simulationen in dem MM-AD umfasst, um Schwellenwertabweichungen des Ausgangsparameters OUT-P, die angeben, dass der Betriebsfehler auftritt, für mindestens zwei verschiedene Zeiträume zu bestimmen.

5. Ein Verfahren nach Anspruch 4, wobei Schwellenwertabweichungen des Ausgangsparameters OUT-P, die angeben, dass der Betriebsfehler auftritt, für einen Zeitraum von einem Tag bestimmt werden.

6. Ein Verfahren nach Anspruch 4, wobei Schwellenwertabweichungen des Ausgangsparameters OUT-P, die angeben, dass der Betriebsfehler auftritt, für einen Zeitraum von mehreren aufeinanderfolgenden Tagen bestimmt werden.

7. Ein Verfahren nach einem der vorhergehenden Ansprüche, wobei das Auswählen eines Ausgabeparameters OUT-P, der mit einer Variation des Betriebsparameters variiert, das Durchführen einer Empfindlichkeitsanalyse von Ausgabeparametern umfasst.

8. Ein Verfahren nach Anspruch 7, wobei die Empfindlichkeitsanalyse Folgendes umfasst:

   - auswählen von mehreren Ausgangsparametern, die mit einer Variation des Betriebsparameters variieren können;
   - simulieren der Variation jedes Ausgabeparameters über einen vorbestimmten Zeitraum, wenn der Betriebsparameter in Bezug auf einen beim Faulbehälterbetrieb zu verwendenden Nennwert variiert; und
   - vergleichen der Variationen der Vielzahl von Ausgangsparametern über den vorbestimmten Zeitraum für die gleiche Variation des Betriebsparameters; und
   - auswählen von mindestens einem Ausgabeparameter, bei dem die zeitliche Variation höher ist als bei anderen Ausgabeparametern.

9. Ein Verfahren nach einem der vorhergehenden Ansprüche, wobei die Vorbereitungsstufe das Kalibrieren der kinetischen Parameter des MM-AD eines anaeroben Faulbehälters, in dem Fehler zu diagnostizieren sind, umfasst.

10. Ein Verfahren nach einem der vorhergehenden Ansprüche, wobei die Betriebsstufe das Kalibrieren der kinetischen Parameter des MM-AD des anaeroben Faulbehälters umfasst.

11. Ein Verfahren nach einem der vorhergehenden Ansprüche, wobei mindestens ein ausgewählter Ausgabeparameter der Biogasausgabestrom des anaeroben Faulbehälters und/oder der Methanausgabestrom des anaeroben Faulbehälters ist.

12. Ein System zum Diagnostizieren von Betriebsfehlern während des Betriebs eines anaeroben Faulbehälters (10), wobei das System Folgendes umfasst:

   - ein Computersystem (70) mit einem mathematischen Modell der anaeroben Faulung (MM-AD) und
   - einen Prozessor (60), der konfiguriert ist, um das Verfahren nach einem der Ansprüche 1 bis 11 durchzuführen.

13. Ein anaerobes Faulbehältersystem, umfassend:

   - ein anaerober Faulbehälter (10);
   - Sensoren (50) und/oder ein Probensammelsystem, um Messungen von Ausgabeparametern während des Betriebs des anaeroben Faulbehälters (60) zu erhalten; und
   - ein System zum Diagnostizieren von Betriebsfehlern während des Betriebs des anaeroben Faulbehälters nach Anspruch 12.

**Revendications**

1. Un procédé pour diagnostiquer des défauts de fonctionnement pendant le fonctionnement d'un digesteur anaérobie (10), comprenant :

   - une étape de préparation, dans laquelle un modèle mathématique de digestion anaérobie (MM-AD) est exécuté dans un système informatique, l'étape de préparation comprenant :

      - sélectionner (210) un défaut de fonctionnement à diagnostiquer ;
      - sélectionner (210) un paramètre de fonctionnement OP-P qui est associé au défaut de fonctionnement sélectionné ; et
      - exécuter (220) des simulations multiples dans le MM-AD pour :

         - sélectionner (230, 240) un paramètre de sortie OUT-P qui varie avec une variation du paramètre de fonctionnement OP-P ; et
         - déterminer (250, 260, 270, 280) une déviation de seuil du paramètre de sortie OUT-P qui indique que le défaut de fonctionnement se produit ; et

   après l'étape de préparation :
   - une étape de fonctionnement de digesteur, dans laquelle un modèle mathématique de digestion anaérobie (MM-AD) est exécuté dans un système informatique, en parallèle avec le fonctionnement physique du digesteur anaérobie, pour simuler le fonctionnement du digesteur au fil du temps, l'étape de fonctionnement du digesteur comprenant :

- saisir dans le MM-AD des valeurs nominales de paramètres cinétiques et de paramètres de fonctionnement du digesteur ;
- démarrer (410, 420) le fonctionnement du digesteur et la simulation basée sur le MM-AD ;
- obtenir (430) au fil du temps des valeurs mesurées MV du paramètre de sortie OUT-P du digesteur, à l'aide de capteurs et/ou en effectuant une analyse d'échantillon ;
- obtenir (440) au fil du temps des valeurs attendues EV du paramètre de sortie OUT-P, telles que fournies par le MM-AD ;
- comparer (450) les valeurs mesurées MV et les valeurs attendues EV du paramètre de sortie OUT-P, au fil du temps ;
- déterminer (460) que le défaut de fonctionnement se produit si la déviation entre la valeur mesurée MV et la valeur attendue EV du paramètre de sortie OUT-P est supérieure à la déviation de seuil déterminée dans l'étape de préparation.

2. Un procédé selon la revendication 1, dans lequel l'étape de préparation comprend sélectionner plus d'un défaut de fonctionnement à diagnostiquer et, pour chaque défaut de fonctionnement :

- sélectionner (210) un paramètre de fonctionnement OP-P qui est associé au défaut de fonctionnement sélectionné ; et
- exécuter (220) des simulations multiples dans le MM-AD, de manière à :

- pour chaque paramètre de fonctionnement sélectionné OP-P, sélectionner (230, 240) un paramètre de sortie OUT-P qui varie avec une variation du paramètre de fonctionnement correspondant OP-P ; et
- pour chaque paramètre de sortie sélectionné OUT-P, déterminer (250, 260, 270, 280) une déviation de seuil qui indique que le défaut de fonctionnement sélectionné se produit.

3. Un procédé selon la revendication 2, comprenant en outre, après l'étape de préparation :

- une étape de fonctionnement de digesteur, dans laquelle un modèle mathématique de digestion anaérobie (MM-AD) est exécuté dans un système informatique, en parallèle avec le fonctionnement physique du digesteur anaérobie, pour simuler le fonctionnement du digesteur au fil du temps, l'étape de fonctionnement du digesteur comprenant :

- saisir dans le MM-AD les valeurs nominales des paramètres cinétiques et des paramètres de fonctionnement du digesteur ;
- démarrer (410, 420) le fonctionnement du digesteur et la simulation basée sur le MM-AD ;
- obtenir (430) au fil du temps des valeurs mesurées MV du paramètre de sortie OUT-P du digesteur, à l'aide de capteurs et/ou en effectuant une analyse d'échantillon ;
- obtenir (440) et comparer (450) au fil du temps les valeurs mesurées MV et les valeurs attendues EV de chaque paramètre de sortie OUT-P sélectionné, et
- déterminer (460) que l'un des défauts de fonctionnement sélectionnés se produit si la déviation entre la valeur mesurée MV et la valeur attendue EV de l'un des paramètres de sortie OUT-P sélectionnés est supérieure à la déviation de seuil correspondant déterminée dans l'étape de préparation.

4. Un procédé selon l'une quelconque des revendications précédentes, dans lequel l'étape de préparation comprend l'exécution (220) de simulations multiples dans le MM-AD pour déterminer des déviations de seuil du paramètre de sortie OUT-P qui indiquent que le défaut de fonctionnement se produit, pendant au moins deux périodes de temps différentes.

5. Un procédé selon la revendication 4, dans lequel les déviations de seuil du paramètre de sortie OUT-P qui indiquent que le défaut de fonctionnement se produit, sont déterminées pendant une période de temps d'un jour.

6. Un procédé selon la revendication 4, dans lequel les déviations de seuil du paramètre de sortie OUT-P qui indiquent que le défaut de fonctionnement se produit, sont déterminées pendant une période de temps de plusieurs jours consécutifs.

7. Un procédé selon l'une quelconque des revendications précédentes, dans lequel la sélection d'un paramètre de sortie OUT-P qui varie avec une variation du paramètre de fonctionnement comprend la réalisation d'une analyse de sensibilité de paramètres de sortie.

8. Un procédé selon la revendication 7, dans lequel l'analyse de sensibilité comprend :

- sélectionner plusieurs paramètres de sortie qui peuvent varier avec une variation du paramètre de fonctionnement ;
- simuler la variation de chaque paramètre de sortie, sur une période de temps prédéterminée, lorsque le paramètre de fonctionnement varie par rapport à une valeur nominale à utiliser dans le fonctionnement du digesteur ; et
- comparer les variations sur la période de temps prédéterminée de la pluralité de paramètres de sortie, pour la même variation du paramètre de fonctionnement ; et
- sélectionner au moins un paramètre de sortie pour lequel la variation au fil du temps est plus élevée que pour d'autres paramètres de sortie.

9. Un procédé selon l'une quelconque des revendications précédentes, dans lequel l'étape de préparation comprend l'étalonnage des paramètres cinétiques du MM-AD d'un digesteur anaérobie dans lequel des défauts doivent être diagnostiqués.

10. Un procédé selon l'une quelconque des revendications précédentes, dans lequel l'étape de fonctionnement comprend l'étalonnage des paramètres cinétiques du MM-AD du digesteur anaérobie.

11. Un procédé selon l'une quelconque des revendications précédentes, dans lequel au moins un paramètre de sortie sélectionné est le flux de sortie de biogaz du digesteur anaérobie, et/ou le flux de sortie de méthane du digesteur anaérobie.

12. Un système pour diagnostiquer des défauts de fonctionnement pendant le fonctionnement d'un digesteur anaérobie (10), le système comprenant :

- un système informatique (70) avec un modèle mathématique de digestion anaérobie (MM-AD), et
- un processeur (60) configuré pour exécuter le procédé de l'une quelconque des revendications 1 à 11.

13. Un système de digesteur anaérobie, comprenant :

- un digesteur anaérobie (10) ;
- des capteurs (50), et/ou un système de collecte d'échantillons, pour obtenir des mesures de paramètres de sortie pendant le fonctionnement du digesteur anaérobie (60) ; et
- un système pour diagnostiquer des défauts de fonctionnement pendant le fonctionnement du digesteur anaérobie, selon la revendication 12.

# FIGURE 1

**FIGURE 2**

```
┌─────────────────────────┐
│     SELECT FAULT AND     │── 210
│     ASSOCIATED OP-P      │
└─────────────────────────┘
             ↓
┌─────────────────────────┐
│   PERFORM SIMULATIONS    │── 220
│    FOR VALUES OF OP-P    │
└─────────────────────────┘
             ↓
┌─────────────────────────┐
│     OBTAIN VALUES OF     │── 230
│      SEVERAL OUT-P       │
└─────────────────────────┘
             ↓
┌─────────────────────────┐
│    SELECT SENSITIVE AND  │── 240
│     MEASURABLE OUT-P     │
└─────────────────────────┘
             ↓
┌─────────────────────────┐
│  DETERMINE RV (OUT-P)    │
│    CORRESPONDING TO      │── 250
│  INTENDED NOMINAL OP-P   │
└─────────────────────────┘
             ↓
┌─────────────────────────┐
│   SELECT %Δ (OP-P)       │── 260
│    INDICATING FAULT      │
└─────────────────────────┘
             ↓
┌─────────────────────────┐
│  DETERMINE %Δ (OUT-P)    │── 270
│ CORRESPONDING TO %Δ (OP-P)│
└─────────────────────────┘
             ↓
┌─────────────────────────┐
│    SET TD = %Δ (OUT-P)   │── 280
└─────────────────────────┘
```

# FIGURE 3

# FIGURE 4

# FIGURE 5

# FIGURE 6

**EP 4 424 810 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013138690 A1

- US 2009048816 A1

### Non-patent literature cited in the description

- Modeling the dynamic performance of full-scale anaerobic primary sludge digester using Anaerobic Digestion Model No. 1 (ADM1). **ERSAHIN MUSTAFA EVREN ED - MORENO JAIME A et al.** BIOPROCESS ANO BIOSYSTEMS ENGINEERING. SPRINGER, 12 July 2018, vol. 41, 1539-1545
- Nonlinear model predictive control of anaerobic digestion process based on reduced ADM1. **XUE LEI et al.** 2015 10TH ASIAN CONTROL CONFERENCE (ASCC). IEEE, 31 May 2015, 1-6

- **ORDACE A et al.** Predictive control of anaerobic digestion of wastewater sludge. A feasibility study. *2012 16TH INTERNATIONAL CONFERENCE ON SYSTEM THEORY, CONTROL ANO COMPUTING*, 12 October 2012, 1-7
- **KAZEMI PEZHAM ER AL**. Data-driven techniques for fault detection in an anaerobic digestion process. *PROCESS SAFETY AND ENVIRONMENTAL PROTETCTION, INSTITUTION OF CHEMICAL ENGINEERS*, 24 December 2020, vol. 146, 905-915